# EUROPEAN PATENT SPECIFICATION

(11) **EP 3 145 481 B1**
(45) Date of publication and mention of the grant of the patent: **19.04.2023**
(21) Application number: 15795676.4
(22) Date of filing: 21.05.2015
(51) Int. Cl.: A61K 8/36, A61K 8/44, A61K 8/46, A61Q 5/02, A61Q 19/10

(54) **SULFATE-FREE PERSONAL CARE COMPOSITIONS**
SULFATFREIE KÖRPERPFLEGEZUSAMMENSETZUNGEN
COMPOSITIONS DE SOIN PERSONNEL SANS SULFATES

(30) Priority: 21.05.2014 US 201462001112 P
(43) Date of publication of application: 29.03.2017
(73) Proprietor: RHODIA OPERATIONS, 69003 Lyon (FR)
(72) Inventor: KIPLINGER, Jon, Newtown, PA 18940 (US); DOBROWOLSKI, David, V., Robbinsville, NJ 08691 (US); PATEL, Rajesh, Pennington, NJ 08534 (US)
(74) Representative: Lederer & Keller Patentanwälte Partnerschaft mbB
(86) International application number: PCT/US2015/031901
(87) International publication number: WO 2015/179599

(56) References cited:
- WO-A1-2005/110356
- WO-A2-2015/071298
- US-A1- 2011 245 125
- US-A1- 2011 275 552
- US-A1- 2012 010 115
- US-A1- 2012 094 884
- US-A1- 2013 210 696
- US-B1- 8 114 824
- US-B2- 7 776 318
- US-B2- 8 227 393
- DATABASE GNPD [Online] MINTEL; 12 February 2014 (2014-02-12), anonymous: "Moisturising Facial Cleanser", XP55557752, retrieved from www.gnpd.com Database accession no. 2313586
- DATABASE GNPD [Online] MINTEL; 16 September 2013 (2013-09-16), anonymous: "Cleanser", XP055557755, retrieved from www.gnpd.com Database accession no. 2175412

## Description

### CROSS-REFERENCE TO RELATED APPLICATIONS

### FIELD OF INVENTION

**This** invention relates to the field of sulfate-free or substantially sulfate-free personal care compositions such as body washes, shampoos, hand soaps, and the like.

### BACKGROUND

Many commercially available personal care compositions, such as body washes, shampoos, hand soaps, etc. are based on sulfate-containing surfactants such as SLS (sodium lauryl sulfate) or SLES (sodium laureth sulfate). While these surfactants are have good foaming properties and can be thickened easily, there have been growing concerns in the marketplace over the negative effects of these and other sulfate-containing surfactants on the skin and body. For example, SLS is widely regarded as a potential irritant for skin.

Conventional sulfate-free personal care compositions are comprised of water, surfactants, vegetable oils, fragrance, and other minor ingredients such as preservatives, additives, and pigments. It is well known in the industry, however, that most sulfate free personal care formulations foam poorly, may contain potentially irritating surfactants, are opaque, and are hard to thicken without the use of polyacrylate rheology modifiers. Compared to traditional salt thickening mechanisms related to alkyl and alkyl ether sulfates, commonly used to create adequately foaming formulations, typical sulfate-free formulations foam less and are thickened by using acrylate polymers and acrylate crosspolymers.

Accordingly, there is an increasing demand for sustainable, safe and/or environment friendly personal care products that are sulfate-free or substantially sulfate-free, but which have improved properties over conventional sulfate-free formulations.

### SUMMARY

This object, and others which will become apparent from the following detailed description, are met by the present invention which is defined in claim 1.

The resulting personal care formulation foams well, and is clear and mild. The composition is thickened without the use of polyacrylates. the w/w ratio A/B is comprised between about 2 and 6. In other words, the weight ratio of A to B falls anywhere between about 2 and about 6, respectively. In another embodiment, the w/w ratio A/B is comprised between about 3 and 5. In another embodiment, the w/w ratio A/B is comprised between about 3.5 and 4.5. In another embodiment, the w/w ratio of A/B is between about 2.5 and 5.5. In another embodiment, the w/w ratio of A/B is between about 3.5 and 5.

In one embodiment, the surfactant component comprises greater than about 10% by weight of the personal care composition.

The alkanoyl glycinate or acyl glycinate is, in another embodiment, of formula (I):

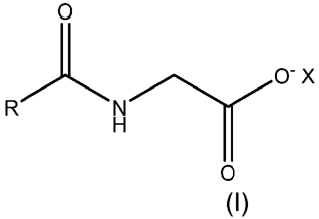

Wherein R is a C₈-C₂₂ alkyl Group, and X is a cation or H. The cation can be selected from sodium, potassium, or ammonium. In other embodiments, R is a C₈-C₁₈ alkyl group, while in further embodiments, R is a C₁₀-C₁₄ group.

In other embodiments, the zwitterionic surfactant or amphoteric surfactant is selected from: alkyl betaines and salts thereof, amidopropyl betaines and salts thereof, alkyl hydroxysulfobetaines and salts thereof, alkyl sulfobetaines and salts thereof, or any combination thereof. The at least one zwitterionic surfactant or amphoteric surfactant, in other embodiments, is selected from cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl)carboxy methyl betaine, stearyl bis-(2-hydroxy-propyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine, alkylamidopropylhydroxy sultaines, such as lauramidopropyl hydroxysultaine, lauryl hydroxysultaine and cocamidopropyl hydroxysultaine, or any combination thereof

**The** personal care composition can be any suitable composition, including but not limited to, shampoos, body washes, hand soaps, lotions, creams, conditioners, shaving products, facial washes, personal wipes, and skin treatments.

It is understood that the personal care composition as described herein can optionally contain other components, including one or more benefit agents.

In one embodiment, the benefit agent comprises one or more compounds selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent-waving, reflectants, essential oils and fragrances.
In another aspect, described herein are methods of preparing sulfate-free personal care compositions, comprising at least the steps of mixing water, (A) at least one of a zwitterionic surfactant or an amphoteric surfactant, (B) at least one alkanoyl glycinate and (C) at least one non-ionic surfactant.

The method for making an aqueous sulfate-free personal care composition, comprises at least the steps of mixing water, at least one the at least one zwitterionic surfactant or amphoteric surfactant, preferably a betaine, at least one glycinate, preferably alkanoyl glycinate (or otherwise synonymously referred to as "acyl glycinate"), and at least one non-ionic surfactant or fatty acid.

The surfactant chassis can be essentially "sulfate free" and "amide free."

### BRIEF DESCRIPTION OF THE DRAWINGS

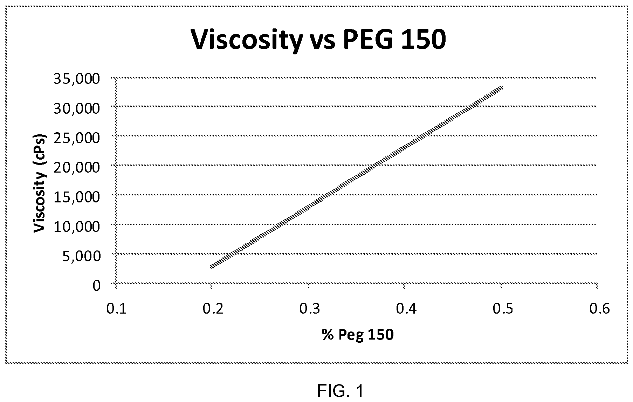

FIG. 1 is a plot of Viscosity versus % PEG (polyethylene glycol) according to one embodiment of the present invention.

### DETAILED DESCRIPTION

As used herein, the term "alkyl" means a saturated straight, branched, or cyclic hydrocarbon radical, such as for example, methyl, ethyl, n-propyl, iso-propyl, n-butyl, sec-butyl, t-butyl, pentyl, n-hexyl, cyclohexyl, decyltetradecyl, octadecyloctadecyl.

As used herein, the term "hydroxyalkyl" means an alkyl radical that is substituted with one or more hydroxyl substituents, such as for example, hydroxyethyl, hydroxypropyl.

As used herein, the term "alkoxyl" means a saturated or unsaturated straight chain or branched chain ether radical, such as for example, ethoxy, propoxy, isopropoxy, butoxy, the term "alkoxylated" or "alkoxylate" in reference to an organic moiety means that the moiety is substituted with one or more alkoxy groups, typically with a polyether group, such as, for example a poly(ethoxy), poly(propoxy) or poly(ethoxypropoxy) group, the term "propoxylated" in reference to an organic moiety means that the moiety is substituted with a at least one propoxyl unit, and the term "butoxylated" in reference to an organic moiety means that the moiety is substituted with at least one butoxyl unit. As used herein, the notation "(n)", wherein n is an integer, in reference to the polyalkoxy group of an alkoxylated moiety indicates the number of alkoxy units in the polyalkoxy group. For example, "propoxylated (5) decyl alcohol" means decyl alcohol alkoxylated with 5 moles of propoxyl units per mole of decyl alcohol and butoxylated (3) dodecyl alcohol means decyl alcohol alkoxylated with 3 moles of butoxyl units per mole of decyl alcohol.

As used herein, the terminology "(Cₙ-Cₘ)" in reference to an organic group, wherein n and m are each integers, indicates that the group may contain from n carbon atoms to m carbon atoms per group.

As used herein, the term "alkylene" means a bivalent straight or branched acyclic saturated hydrocarbon radical, including methylene and polymethylene, such as, for example, dimethylene, tetramethylene, 2-methyltrimethylene.

As used herein, the term "alkenyl" means an unsaturated straight, branched, or cyclic hydrocarbon radical having at least one carbon-carbon double bond per radical, such as for example, propenyl, butenyl.

As used herein, the term "alkyleneoxy" means a bivalent straight or branched acyclic ether or polyether radical such as, for example, ethyleneoxy, poly(ethyleneoxy), propyleneoxy, poly(propyleneoxy), poly(ethoxylenepropyleneoxy).

As used herein, the term "cleansing composition" means a composition aimed to the washing/cleaning, more typically a body-care application, including but not limited to body wash, shower gel, liquid hand soap, shampoo or the like

In one embodiment, the alkanoyl glycinate or acyl glycinate is of formula (I):

RC(O)NHCH₂CO₂X (I),

wherein R is a C₈-C₂₂ alkyl group, and X is a cation or H.

Where X is a cation, the cation can be any suitable cation. In one embodiment, the cation is sodium, potassium, or ammonium. In another embodiment, the cation is lithium, alkyl ammonium, an alkaline earth metal such as calcium or magnesium, triethanolammonium, trialkylammonium, monoalkylammonium, dialkylammonium, isopropylammonium, monoethanolammonium, or diethanolammonium.

The R group is, in one embodiment, a C₈-C₁₈ alkyl group. In another embodiment, R is a C₁₀-C₁₄ alkyl group. In another embodiment, R is a C₁₂-C₁₄ alkyl group. In another embodiment, R is a C₈-C₁₄ group. In another embodiment, R is a C₁₂ group. In another embodiment, R is a C₁₀ group. In another embodiment, R is a C₁₄ group.

In one particular embodiment, the acyl glycinate is cocoyl glycinate or a cocoyl glycinate salt, more typically sodium cocoyl glycinate. In another particular embodiment, the acyl glycinate is lauryl glycinate or a lauryl glycinate salt, more typically, sodium lauryl glycinate. In another embodiment, the acyl glycinate is a mixture of cocoyl glycinate and lauryl glycinate. In another embodiment, the acyl glycinate is a mixture of at least two of: cocoyl glycinate, lauryl glycinate, cocoyl glycinate salt, and/or lauryl glycinate salt.

The at least one zwitterionic surfactant or amphoteric surfactant, in one embodiment, is an alkyl betaine, alkyl sulfobetaine, an alkyl dimethyl betaine, an alkyl amidopropyl hydroxy sulfobetaine or alkyl hydroxy sulfobetaine. In some embodiments, the wherein the alkyl group has an upper limit of 10 carbon atoms, or 12 carbon atoms, or 14 carbon atoms, 16 carbon atoms, 18 carbon atoms, 20 carbon atoms or 22 carbon atoms.

In one embodiment, the sulfobetaine of the present invention is of formula (II): 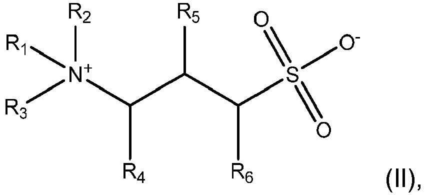 wherein R₁ is an alkylamido group or a linear or branched alkyl group; R₂ and R₃ are individually hydrogen, a methyl group or a hydroxyethyl group; R₄, R₅ and R₆ are individually hydrogen or a hydroxy group. In one embodiment, the alkyl group has greater than about 10 carbon atoms. In one embodiment, the alkyl group has greater than about 11, 12 or 13 carbon atoms. In another embodiment, the alkyl group has greater than about 14 or 16 carbon atoms.

In one embodiment, the alkylamido group has formula (III): 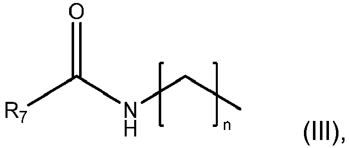 wherein R7 is a linear or branched alkyl group having greater than about 10 carbon atoms, wherein n is an integer from 2 to 5. In one embodiment, "n" is an integer of 3, and in another embodiment "n" is an integer of 4.

In some embodiments, zwitterionic surfactants include but are not limited to compounds having the formula (IIIa): 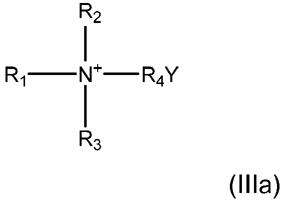 wherein R1 represents a hydrophobic moiety of alkyl, alkylarylalkyl, alkoxyalkyl, alkylaminoalkyl and alkylamidoalkyl, wherein alkyl represents a group that contains from about 10 to about 24 carbon atoms which may be branched or straight chained and which may be saturated or unsaturated. Representative long-chain alkyl groups include tetradecyl (myristyl), hexadecyl (cetyl), octadecenyl (oleyl), octadecyl (stearyl), docosenoic (erucyl) and the derivatives of tallow, coco, soya and rapeseed oils. The typical alkyl groups have from about 16 to about 22 carbon atoms. Representative of alkylamidoalkyl is alkylamidopropyl with alkyl being as described above.

R2 and R3 are independently an aliphatic chain (i.e. as opposed to aromatic at the atom bonded to the quaternary nitrogen, e.g., alkyl, arylalkyl, hydroxyalkyl, carboxyalkyl, and hydroxyalkyl-polyoxyalkylene, e.g. hydroxyethyl-polyoxyethylene or hydroxypropyl-polyoxypropylene) having from 1 to about 50 carbon atoms, in other embodiments from about 1 to about 20 carbon atoms, in other embodiments from about 1 to about 10 carbon atoms and in yet other embodiments from about 1 to about 6 carbon atoms in which the aliphatic group can be branched or straight chained, saturated or unsaturated. Exemplary alkyl chains are methyl, ethyl, preferred arylalkyl is benzyl, and preferred hydroxyalkyls are hydroxyethyl or hydroxypropyl, while preferred carboxyalkyls are acetate and propionate. Exemplary hydroxyalkylpolyoxyalkylenes are hydroxyethyl-polyoxyethylene and hydroxypropyl-polyoxyethylene.

R4 is a hydrocarbyl radical (e.g. alkylene) with chain length 1 to 4. In one embodiment, R4 is a methylene or ethylene group.

Y is COO- or CH(OH)CH2SO3- or SO3-

In another embodiment, zwitterionic surfactants include, for example, those which can be broadly described as derivatives of aliphatic quaternary ammonium, phosphonium, and sulfonium compounds in which the aliphatic radicals can be straight chain or branched and wherein one of the aliphatic substituents contains from about 8 to 18 carbon atoms and one contains an anionic water-solubilizing group such as carboxyl, sulfonate, sulfate, phosphate or phosphonate. Specific examples of suitable zwitterionic surfactants include alkyl betaines, such as cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl)carboxy methyl betaine, stearyl bis-(2-hydroxy-propyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine, and alkylamidopropylhydroxy sultaines.

Specific non-limiting examples of suitable zwitterionic surfactants include the following structures: 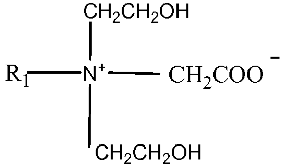 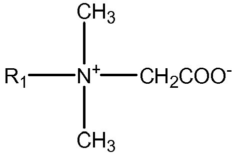 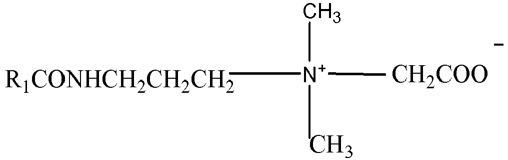 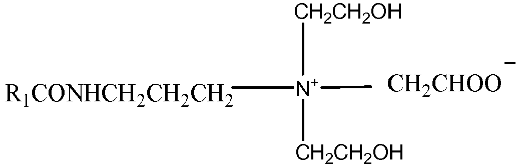 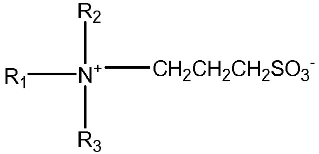 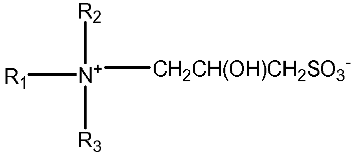 wherein R1 represents a hydrophobic moiety of alkyl, alkylarylalkyl, alkoxyalkyl, alkylaminoalkyl and alkylamidoalkyl, wherein alkyl represents a group that contains from about 10 to about 24 carbon atoms which may be branched or straight chained and which may be saturated or unsaturated. Representative long-chain alkyl groups include tetradecyl (myristyl), hexadecyl (cetyl), octadecenyl (oleyl), octadecyl (stearyl), docosenoic (erucyl) and the derivatives of tallow, coco, soya and rapeseed oils. The typical alkyl groups have from about 16 to about 22 carbon atoms. Representative of alkylamidoalkyl is alkylamidopropyl with alkyl being as described above.

R2 and R3 are independently an aliphatic chain (i.e. as opposed to aromatic at the atom bonded to the quaternary nitrogen, e.g., alkyl, arylalkyl, hydroxyalkyl, carboxyalkyl, and hydroxyalkyl-polyoxyalkylene, e.g. hydroxyethyl-polyoxyethylene or hydroxypropyl-polyoxypropylene) having from 1 to about 50 carbon atoms, in other embodiments from about 1 to about 20 carbon atoms, in other embodiments from about 1 to about 10 carbon atoms and in yet other embodiments from about 1 to about 6 carbon atoms in which the aliphatic group can be branched or straight chained, saturated or unsaturated.

**Optionally,** one or more anionic surfactants can be included in the surfactant component or as an additional surfactant to the personal care compositions as described herein.

Anionic surfactants are known. Any anionic surfactant that is acceptable for use in the intended end use application is suitable as the anionic surfactant component of the composition of the present invention, including, for example, linear alkylbenzene sulfonates, alpha olefin sulfonates, paraffin sulfonates, alkyl ester sulfonates, alkyl sulfates, alkyl alkoxy sulfates, alkyl sulfonates, alkyl alkoxy carboxylates, alkyl alkoxylated sulfates, monoalkyl phosphates, dialkyl phosphates, sarcosinates, sulfosuccinates, isethionates, and taurates, as well as mixtures thereof. Commonly used sulfate free anionic surfactants that are suitable as the anionic surfactant component of the composition of the present invention include, for example, sodiummonoalkyl phosphates, sodium dialkyl phosphates, sodium lauroyl sarcosinate, lauroyl sarcosine, cocoyl sarcosinate, sodium cocoyl isethionate, disodium laureth sulfosuccinate, sodium methyl oleoyl taurate, sodium methyl cocoyl taurate, sodium laureth carboxylate, sodium trideceth carboxylate, Ultra mild amino acid based anionic surfactants include, for example sodium cocoyl glycinate , sodium lauryl glycinate, Sodium Cocoyl Alinate and various glutamates and mixtures thereof.

The cation of any anionic surfactant is typically sodium but may alternatively be potassium, lithium, calcium, magnesium, ammonium, or an alkyl ammonium having up to 6 aliphatic carbon atoms including isopropylammonium, monoethanolammonium, diethanolammonium, and triethanolammonium. Ammonium and ethanolammonium salts are generally more soluble that the sodium salts. Mixtures of the above cations may be used.

The composition of the present invention may optionally further comprise, in addition to the anionic surfactant and amphodiacetate components of the composition of the present invention, one or more cationic surfactants, one or more additional non-ionic surfactants, one or more zwitterionic surfactants, one or more amphoteric surfactants, one or more electrolytes, or a mixture thereof. Cationic surfactants and certain nonionic surfactants, such as such as fatty alcohols, ethoxylated alcohols, and fatty acids, are known to act as structurants for anionic surfactants.

Cationic surfactants are known. Any cationic surfactant that is acceptable for use in the intended end use application is suitable as the cationic surfactant component of the composition of the present invention, including, for example, cationic surfactants according to formula (IV) below: 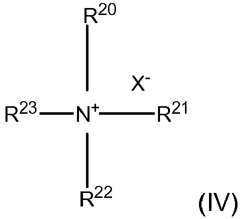 wherein: R²⁰, R²¹, R²², and R²³ are each independently hydrogen or an organic group, provided that at least one of R²⁰, R²¹, R²², and R²³ is not hydrogen, and X⁻ is an anion.

For quaternary ammonium compounds (generally referred to as "quats") R²⁰, R²¹, R²², and R²³ may be the same or different organic group, but may not be hydrogen. In one embodiment, R²⁰, R²¹, R²², and R²³ are each (C₈-C₂₄) branched or linear hydrocarbon groups which may be substituted or interrupted by additional functional moieties and include, for example, fatty acids or derivatives thereof, including esters of fatty acids and fatty acids with alkoxylated groups, alkyl amido groups, aromatic rings, heterocyclic rings, phosphate groups, epoxy groups, and hydroxyl groups. The nitrogen atom may also be part of a heterocyclic or aromatic ring system, e.g., cetethyl morpholinium ethosulfate or steapyrium chloride. Suitable anions include, for example, chloride, bromide, methosulfate, ethosulfate, lactate, saccharinate, acetate or phosphate. Examples of quaternary ammonium compounds of the monoalkyl amine derivative type include: cetyl trimethyl ammonium bromide (also known as CETAB or cetrimonium bromide), cetyl trimethyl ammonium chloride (also known as cetrimonium chloride), myristyl trimethyl ammonium bromide (also known as myrtrimonium bromide or Quaternium-13), stearyl dimethyl benzyl ammonium chloride (also known as stearalkonium chloride), oleyl dimethyl benzyl ammonium chloride, (also known as olealkonium chloride), lauryl/myristryl trimethyl ammonium methosulfate (also known as cocotrimonium methosulfate), cetyl-dimethyl-(2)hydroxyethyl ammonium dihydrogen phosphate (also known as hydroxyethyl cetyldimonium phosphate), bassuamidopropylkonium chloride, cocotrimonium chloride, distearyldimonium chloride, wheat germ-amidopropalkonium chloride, stearyl octyldimonium methosulfate, isostearaminopropal-konium chloride, dihydroxypropyl PEG-5 linoleaminium chloride, PEG-2 stearmonium chloride, Quaternium 18, Quaternium 80, Quaternium 82, Quaternium 84, behentrimonium chloride, dicetyl dimonium chloride, behentrimonium methosulfate, tallow trimonium chloride and behenamidopropyl ethyl dimonium ethosulfate. Quaternary ammonium compound of the dialkyl amine derivative type distearyldimonium chloride, dicetyl dimonium chloride, stearyl octyldimonium methosulfate, dihydrogenated palmoylethyl hydroxyethylmonium methosulfate, dipalmitoylethyl hydroxyethylmonium methosulfate, dioleoylethyl hydroxyethylmonium methosulfate, hydroxypropyl bisstearyldimonium chloride, and mixtures thereof.

Quaternary ammonium compounds of the imidazoline derivative type include, for example, isostearyl benzylimidonium chloride, cocoyl benzyl hydroxyethyl imidazolinium chloride, cocoyl hydroxyethylimidazolinium PG-chloride phosphate, Quaternium 32, and stearyl hydroxyethylimidonium chloride, and mixtures thereof.

Nonionic surfactants are known. Any nonionic surfactant that is acceptable for use in the intended end use application is suitable as the optional nonionic surfactant component of the composition of the present invention, including compounds produced by the condensation of alkylene oxide groups with an organic hydrophobic compound which may be aliphatic or alkyl aromatic in nature. Examples of useful nonionic surfactants include the polyethylene, polypropylene, and polybutylene oxide condensates of alkyl phenols, fatty acid amide surfactants, polyhydroxy fatty acid amide surfactants, amine oxide surfactants, alkyl ethoxylate surfactants, alkanoyl glucose amide surfactants, and alkylpolyglycosides. Specific examples of suitable nonionic surfactants include alkanolamides such as cocamide DEA, cocamide MEA, cocamide MIPA, lauramide DEA, and lauramide MEA, alkyl amine oxides such as lauramine oxide, cocamine oxide, cocamidopropylamine oxide, and lauramidopropylamine oxide, sorbitan laurate, sorbitan distearate, fatty acids or fatty acid esters such as lauric acid, and isostearic acid, fatty alcohols or ethoxylated fatty alcohols such as lauryl alcohol, laureth-4, laureth-7, laureth-9, laureth-40, trideceth alcohol, C11-15 pareth-9, C12-13 Pareth-3, and C14-15 Pareth-11, alkylpolyglucosides such as decyl glucoside, lauryl glucoside, and coco glucoside.

Zwitterionic surfactants are known. Any Zwitterionic surfactant that is acceptable for use in the intended end use application is suitable as the optional Zwitterionic surfactant component of the composition of the present invention, including, for example, those which can be broadly described as derivatives of aliphatic quaternary ammonium, phosphonium, and sulfonium compounds in which the aliphatic radicals can be straight chain or branched and wherein one of the aliphatic substituents contains from about 8 to 18 carbon atoms and one contains an anionic water-solubilizing group such as carboxyl, sulfonate, sulfate, phosphate or phosphonate. Specific examples of suitable Zwitterionic surfactants include alkyl betaines, such as cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxyethyl)carboxy methyl betaine, stearyl bis-(2-hydroxy-propyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine, and alkylamidopropylhydroxy sultaines. Specific examples of suitable Zwitterionic surfactants include alkyl betaines, such as cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine, and alkylamidopropylhydroxy sultaines.

Amphoteric surfactants are known. Any amphoteric surfactant that is acceptable for use in the intended end use application is suitable as the optional amphoteric surfactant component of the composition of the present invention, including, for example, derivatives of aliphatic secondary and tertiary amines in which the aliphatic radical can be straight chain or branched and wherein one of the aliphatic substituents contains from about 8 to about 18 carbon atoms and one contains an anionic water solubilizing group. Specific examples of suitable amphoteric surfactants include the alkali metal, alkaline earth metal, ammonium or substituted ammonium salts of alkyl amphocarboxy glycinates and alkyl amphocarboxypropionates, alkyl amphodipropionates, alkyl amphodiacetates, alkyl amphoglycinates, and alkyl amphopropionates, as well as alkyl iminopropionates, alkyl iminodipropionates, and alkyl amphopropylsulfonates , such as for example, cocoamphoacetate cocoamphopropionate, cocoamphodiacetate, lauroamphoacetate, lauroamphodiacetate , lauroamphodipropionate, lauroamphodiacetate, cocoamphopropyl sulfonate caproamphodiacetate, caproamphoacetate, caproamphodipropionate, and stearoamphoacetate.

Any fatty acid that is acceptable for use in the intended application is suitable, including but limited to lauric acid, stearic acid, behenyl acid, coconut fatty acid, oleic acid, myristic acid, or any combinations thereof.

Electrolytes suitable as an additional structurant component of the composition of the present invention include salts of multivalent anions, such as potassium pyrophosphate, potassium tripolyphosphate, and sodium or potassium citrate, salts of multivalent cations, including alkaline earth metal salts such as calcium chloride and calcium bromide, as well as zinc halides, barium chloride and calcium nitrate, salts of monovalent cations with monovalent anions, including alkali metal or ammonium halides, such as potassium chloride, sodium chloride, potassium iodide, sodium bromide, and ammonium bromide, alkali metal or ammonium nitrates, and polyelectrolytes, such as uncapped polyacrylates, polymaleates, or polycarboxylates, lignin sulphonates or naphthalene sulphonate formaldehyde copolymers. Electrolytes may be added as a separate component of the composition or may be added as a part of another component of the composition, e.g., amphoteric surfactants, such as sodium lauroamphoacetate, typically contain an electrolyte, such as sodium chloride.

The composition of the present invention may optionally further comprise one or more polymers and/or thickeners, chosen from the groups of clays, substituted or unsubstituted hydrocolloids, such as, for example, Some examples of clays include bentonite, kaolin, montmorillonite, sodium magnesium silicate, hectorite, magnesium aluminum silicate. Some examples hydrocolloids in the unmodified form include agar, alginate, arabinoxylan, carrageenan, cellulose derivatives, such as carboxyalkyl celluose, hydroxyalkyl cellulose, hydroxyalkyl alkyl cellulose, and alkyl cellulose, curdlan, gelatin, gellan, β-glucan, guar gum, gum arabic, locust bean gum, pectin, starch, succinoglycan, Xanthan gum. Some examples of modified or substituted hydrocolloids are cellulose derivatives, such as carboxyalkyl cellulose, hydroxyalkyl cellulose, hydroxyalkyl alkyl cellulose, alkyl cellulose, hydroxy methyl cellulose, PG-hydroxyethyl cellulose, quaternary ammonium derivatives of hydroxyethyl cellulose, quaternary ammonium derivatives of guar gum (such as Jaguar C-17, Jaguar C-14S, Jaguar Excel, Jaguar C-162 from Rhodia), hydroxypropyl guars (Jaguar HP-8, Jaguar HP-105, Jaguar HP-60, Jaguar HP-120, Jaguar C-162), modified starches, such as sodium hydroxypropyl starch phosphate (Pure-Gel 980 and Pure-Gel 998 from Grain Processing Corporation), potato starch modified (such as Structure-Solanace from National Starch), cationic polymers (such as Rheovis CSP, Rheovis CDE, Rheovis CDP from Ciba), hydrohobically modified nonionic polyols (Acusol 880, Acusol 882 from Rohm & Hass). In general, personal care compositions may optionally comprise, based on 100 pbw of the personal care composition and independently for each such ingredient, up to about 10 pbw, typically from about 0.1 pbw to about 5.0 pbw, more typically from about 0.5 pbw to about 4.0 pbw, of such other ingredients, depending on the desired properties of the personal care composition.

In one embodiment, the composition of the present invention further comprises one or more water insoluble or partially water soluble components. Such components may be in the form of a solid, a liquid, or a gas and may comprise one or more materials selected from water insoluble or partially water soluble benefit agents, such as, for example, in the case of a personal care application, emollients, conditioners, moisturizers, vitamins, vitamin derivatives, moisturizing beads, natural or synthetic abrasives, such as polyoxyethylene beads, anti-UV agents, anti-bacterial agents, antifungal agents, tanning accelerators, anti-aging agents, anti-wrinkle agents, antiperspirants, deodorants, essential oils, fragrances, air, or abrasives, and water insoluble or partially water soluble chemically stable appearance modifying additives such as, for example, pigments, opacifying agents, colored or reflective particles or beads such as particles of mica, titanium dioxide, or glycol stearate. It is preferred that the benefit agents are chemically stable in the chosen surfactant system.

The composition of the present invention is useful in, for example, personal care applications, such as shampoos, body wash, hand soap, lotions, creams, conditioners, shaving products, facial washes, neutralizing shampoos, personal wipes, and skin treatments, and in home care applications, such as liquid detergents, laundry detergents, hard surface cleansers, dish wash liquids, toilet bowl cleaners, as well as other applications, such as oil field and agrochemical applications.

In one embodiment, the composition of the present invention is a personal care composition.

In one embodiment, the personal care composition of the present invention comprises a surfactant component of the present invention in combination with additional water and/or one or more additional ingredients and suitable personal care compositions are made by diluting the composition with water and/or mixing a structured surfactant composition with additional ingredients.

In one embodiment, the personal care composition of the present invention further comprises one or more benefit agents, such as emollients, moisturizers, conditioners, skin conditioners, or hair conditioners such as vegetable oils, including arachis oil, castor oil, cocoa butter, coconut oil, corn oil, cotton seed oil, olive oil, palm kernel oil, rapeseed oil, safflower seed oil, sesame seed oil and soybean oil, esters, including butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, and propylene glycol isostearate, animal fats, including acetylated lanolin alcohols, lanolin, lard, mink oil and tallow, and fatty acids and alcohols, including behenic acid, palmitic acid, stearic acid, behenyl alcohol, cetyl alcohol, eicosanyl alcohol and isocetyl alcohol; vitamins or their derivatives, such as vitamin B complex, including thiamine, nicotinic acid, biotin, pantothenic acid, choline, riboflavin, vitamin B6, vitamin B12, pyridoxine, inositol, carnitine, vitamins A,C,D,E,K and their derivatives, such as vitamin A palmitate, and pro-vitamins, e.g., panthenol (pro vitamin B5), panthenol triacetate and mixtures thereof; antioxidants; free-radical scavengers; abrasives, natural or synthetic; dyes; hair coloring agents; bleaching agents; hair bleaching agents; UV absorbers, such as benzophenone, bornelone, PABA (Para Amino Benzoic Acid), butyl PABA, cinnamidopropyl trimethyl ammonium chloride, disodium distyrylbiphenyl disulfonate, potassium methoxycinnamate; anti-UV agents, such as butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, octyl dimethyl PABA (padimate O), red petrolatum; antimicrobial agents; antibacterial agents, such as bacitracin, erythromycin, triclosan, neomycin, tetracycline, chlortetracycline, benzethonium chloride, phenol, parachlorometa xylenol (PCMX), triclocarban (TCC), chlorhexidine gluconate (CHG), zinc pyrithione, selenium sulfide; antifungal agents; melanin regulators; tanning accelerators; depigmenting agents, such as retinoids such as retinol, kojic acid and its derivatives such as, for example, kojic dipalmitate, hydroquinone and its derivatives such as arbutin, transexamic acid, vitamins such as niacin, vitamin C and its derivatives, azelaic acid, placertia, licorice, extracts such as chamomile and green tea, where retinol, kojic acid, and hydroquinone are preferred; skin lightening agents such as hydroquinone, catechol and its derivatives, ascorbic acid and its derivatives; skin-coloring agents, such as dihydroxyacetone; liporegulators; weight-reduction agents; anti-acne agents; antiseborrhoeic agents; anti-ageing agents; anti-wrinkle agents; keratolytic agents; anti-inflammatory agents; anti-acne agents, such as tretinoin, isotretinoin, motretinide, adapalene, tazarotene, azelaic acid, retinol, salicylic acid, benzoyl peroxide, resorcinol, antibiotics such as tetracycline and isomers thereof, erythromycin, anti-inflammatory agents such as ibuprofen, naproxen, hetprofen, botanical extracts such as alnus, amica, artemisia capillaris, asiasarum root, calendula, chamomile. cnidium, comfrey, fennel, galla rhois, hawthorn, houttuynia, hypericum, jujube, kiwi, licorice, magnolia, olive, peppermint, philodendron, salvia, sasa albomarginata, imidazoles such as ketoconazole and elubiol, those anti-acne agents described in Gollnick, H. et al. 196(I) Dermatology Sebaceous Glands, Acne and Related Disorders, 119-157 (1998), which is incorporated by reference herein to the extent that it is not inconsistent with the present application; refreshing agents; cicatrizing agents; vascular-protection agents; agents for the reduction of dandruff, seborrheic dermatitis, or psoriasis, such as zinc pyrithione, shale oil and derivatives thereof such as sulfonated shale oil, selenium sulfide, sulfur, salicylic acid, coal tar, povidone-iodine, imidazoles such as ketoconazole, dichlorophenyl imidazolodioxalan, clotrimazole, itraconazole, miconazole, climbazole, tioconazole, sulconazole, butoconazole, fluconazole, miconazolenitrite and any possible stereo isomers and derivatives thereof such as anthralin, piroctone olamine (Octopirox), selenium sulfide, ciclopirox olamine, anti-psoriasis agents such as vitamin D analogs, e.g. calcipotriol, calcitriol, and tacaleitrol, vitamin A analogs such as esters of vitamin A including vitamin A palmitate, retinoids, retinols, and retinoic acid, corticosteroids such as hydrocortisone, clobetasone, butyrate, clobetasol propionate; antiperspirants or deodorants, such as aluminum chlorohydrates, aluminum zirconium chlorohydrates; immunomodulators; nourishing agents; depilating agents, such as calcium thioglycolate, magnesium thioglycolate, potassium thioglycolate, strontium thioglycolate; agents for combating hair loss; reducing agents for permanent-waving; reflectants, such as mica, alumina, calcium silicate, glycol dioleate, glycol distearate, silica, sodium magnesium fluorosilicate; essential oils and fragrances.

In one embodiment, the surfactant composition of the present invention comprises a benefit agent selected from insoluble or partially insoluble ingredients such as moisturizers or conditioners, hair coloring agents, anti-UV agents, anti-wrinkle agents, fragrances or essential oils, skin-coloring agents, anti-dandruff agents, and provides enhanced deposition of such benefit agent on the substrate, ex. hair and/or skin or fabric or counter top or plant leaves.

In one embodiment, the personal care composition of the present invention further comprises from about 0.1 to about 50 pbw, more typically from about 0.3 to about 25 pbw, and still more typically from about 0.5 to 10 pbw, of one or more benefit agents.

The personal care composition according to the present invention may optionally further comprise other ingredients, such as, for example, preservatives such as benzyl alcohol, methyl paraben, propyl paraben and imidazolidinyl urea, thickeners and viscosity modifiers such as block polymers of ethylene oxide and propylene oxide, electrolytes, such as sodium chloride, sodium sulfate, and polyvinyl alcohol, pH adjusting agents such as citric acid, succinic acid, phosphoric acid, sodium hydroxide, and sodium carbonate, perfumes, dyes, and sequestering agents, such as disodium ethylenediamine tetra-acetate. In general, personal care compositions may optionally comprise, based on 100 pbw of the personal care composition and independently for each such ingredient, up to about 10 pbw, preferably from 0.5 pbw to about 5.0 pbw, of such other ingredients, depending on the desired properties of the personal care composition.

In general, personal care composition of the present invention may optionally comprise, based on 100 pbw of the personal care composition and independently for each such ingredient, up to about 15 pbw, preferably from 0.5 pbw to about 10 pbw, of such other ingredients, depending on the desired properties of the personal care composition.

The personal care composition of the present invention is used in a manner know in the art, for example, in the case of a cleanser or shampoo, by application of the cleanser or shampoo to the skin and/or hair and optionally rinsing the cleanser or shampoo off of the skin and/or hair with water.

The composition of the present invention is useful in, for example, personal care applications, such as shampoos, body wash, hand soap, lotions, creams, conditioners, shaving products, facial washes, neutralizing shampoos, personal wipes, and skin treatments, and in home care applications, such as liquid detergents, laundry detergents, hard surface cleansers, dish wash liquids, toilet bowl cleaners, as well as other applications, such as oil field and agrochemical applications.

### EXAMPLES

In the following examples all parts and percentages are by weight unless otherwise indicated. The surfactant system as described herein are clear, mild, foam well and may be thickened without the use of acrylate polymers and crosspolymers. In one embodiment, the surfactant systems described herein are tertiary surfactant systems.

### Examples

Sulfate free compositions as described in the Examples herein were prepared using at least the ingredients listed in Tables I-V. Physical properties were measured with the results as set forth below, including pH and viscosity. The viscosity of each of the compositions of Examples 1, 2, 3, 4 and 5 was measured in centipoise (CPS) and using a Brookfield LVF Viscometer equipped with a SP # 3 spindle at 12 revolutions per minute at 25°C for 1 min.

### Example #1, Sulfate Free Foam Wash (not claimed)

**Table I**

| Ingredient | % wt (actives) |
|---|---|
| Lauramidopropyl Betaine | 8% |
| Sodium Cocoyl Glycinate | 2% |
| Lauric Acid | 1% |
| Fragrance | 1% |
| Preservative | 0.10% |
| Water | 87.9% |

| | |
|---|---|
| pH = 6.70, Viscosity = <1000 CPS | |

### Example #2: Sulfate Free Hand Soap/Body Wash

**Table II**

| Ingredient | % wt (actives) |
|---|---|
| Lauramidopropyl Betaine | 8% |
| Sodium Cocoyl Glycinate | 2% |
| Lauric Acid | 2% |
| PEG 150 Distearate | 0.2% |
| Fragrance | 1.00% |
| Preservative | 0.10% |
| Water | 86.7% |

| | |
|---|---|
| pH = 6.80, Viscosity = 2,800 CPS | |

Referring to FIG. 1, the viscosity response as a function of PEG concentration of Example #2 is illustrated.

### Example #3: Sulfate Free Hand Soap/Body Wash (components add up to more than 100%)

**Table III**

| Ingredient | % wt (actives) |
|---|---|
| Cocamidopropyl Betaine | 8% |
| Sodium Cocoyl Glycinate | 2% |
| Lauric Acid | 2% |
| PEG 150 Distearate | 0.5% |
| Fragrance | 1.00% |
| Preservative | 0.10% |
| Water | 86.7% |

| | |
|---|---|
| pH = 6.50, Viscosity = 16,400 CPS | |

### Example #4: Sulfate Free Hand Soap/Body Wash (components add up to less than 100%)

**Table IV**

| Ingredient | % wt (actives) |
|---|---|
| Cocamidopropyl Hydroxysultaine | 8% |
| Sodium Cocoyl Glycinate | 2% |
| Lauric Acid | 0.5% |
| PEG 150 Distearate | 1% |
| Preservative | 0.10% |
| Water | 71.2% |

| | |
|---|---|
| pH = 6.40, Viscosity = 6,160 CPS | |

## Claims

1. An aqueous sulfate-free personal care composition, comprising,
PEG 150 distearate,
a surfactant component comprising:
(A) at least one of a zwitterionic surfactant or an amphoteric surfactant;
(B) at least one alkanoyl glycinate; and
(C) lauric acid,
wherein the w/w ratio A/B is comprised between 2 and 6; and
water,
wherein the composition comprises a minimum of 2 % lauric acid and 0.1 % to 0.5 % PEG 150 distearate and has a pH range of 6.5 to 7.0 and a viscosity ranging from 2 to 30 Pa·s (2,000 to 30,000 centipoise), but does not comprise polyacrylates.

2. The composition of claim 1 wherein the surfactant component comprises greater than 10% by weight of the personal care composition.

3. The composition of claim 1 wherein the w/w ratio A/B is comprised between 3 and 5, preferably between 3.5 and 4.5.

4. The composition of claim 1 wherein the alkanoyl glycinate is of formula (I): 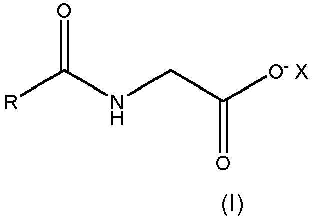 wherein R is a C₈-C₂₂ alkyl group, and X is a cation or H.

5. The composition of claim 4 wherein the cation is selected from sodium, potassium, or ammonium.

6. The composition of claim 4 wherein R is a C₈-C₁₈ alkyl group, preferably a C₁₀-C₁₄ alkyl group.

7. The composition of claim 1 wherein the at least one zwitterionic surfactant or amphoteric surfactant is selected from: alkyl betaines and salts thereof, amidopropyl betaines and salts thereof, alkyl hydroxysulfobetaines and salts thereof, alkyl sulfobetaines and salts thereof, or any combination thereof.

8. The composition of claim 1 wherein the at least one zwitterionic surfactant or amphoteric surfactant is selected from cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl)carboxy methyl betaine, stearyl bis-(2-hydroxy-propyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine, alkylamidopropylhydroxy sultaines, or any combination thereof.

9. The composition of claim 8 wherein the at least one zwitterionic surfactant or amphoteric surfactant comprises an alkylamidopropylhydroxy sultaine selected from lauramidopropyl hydroxysultaine, lauryl hydroxysultaine and cocamidopropyl hydroxysultaine.

10. The composition of claim 1 wherein the composition is selected from shampoos, body washes, hand soaps, lotions, creams, conditioners, shaving products, facial washes, personal wipes, and skin treatments.

11. The composition of claim 1 further comprising one or more benefit agents.

12. The composition of claim 11, wherein the benefit agent comprises one or more compounds selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent-waving, reflectants, essential oils and fragrances.

## Patentansprüche

1. Wässrige sulfatfreie Körperpflegezusammensetzung, umfassend:
PEG150-Distearat,
eine Tensidkomponente umfassend:
(A) wenigstens eines von einem zwitterionischen Tensid und einem amphoteren Tensid;
(B) wenigstens ein Alkanoylglycinat; und
(C) Laurinsäure,
wobei das Gew./Gew.-Verhältnis A/B in dem Bereich zwischen 2 und 6 liegt; und
Wasser,
wobei die Zusammensetzung wenigstens 2 % Laurinsäure und 0,1 % bis 0,5 % PEG150-Distearat umfasst und einen pH-Wert-Bereich von 6,5 bis 7,0 und eine Viskosität in dem Bereich von 2 bis 30 Pa.s (2.000 bis 30.000 Centipoise) aufweist aber keine Polyacrylate umfasst.

2. Zusammensetzung gemäß Anspruch 1, wobei die Tensidkomponente mehr als 10 Gew.-% der Körperpflegezusammensetzung umfasst.

3. Zusammensetzung gemäß Anspruch 1, wobei das Gew./Gew.-Verhältnis A/B in dem Bereich zwischen 3 und 5, vorzugsweise zwischen 3,5 und 4,5, liegt.

4. Zusammensetzung gemäß Anspruch 1, wobei das Alkanoylglycinat von der Formel (I) ist: 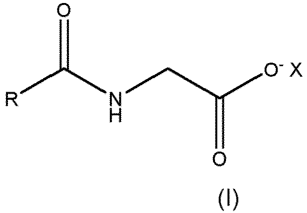 wobei R eine C₈-C₂₂-Alkylgruppe ist und X ein Kation oder H ist.

5. Zusammensetzung gemäß Anspruch 4, wobei das Kation ausgewählt ist aus Natrium, Kalium und Ammonium.

6. Zusammensetzung gemäß Anspruch 4, wobei R eine C₈-C₁₈-Alkylgruppe, vorzugsweise eine C₁₀-C₁₄-Alkylgruppe, ist.

7. Zusammensetzung gemäß Anspruch 1, wobei das wenigstens eine zwitterionische Tensid oder amphotere Tensid ausgewählt ist aus: Alkylbetainen und Salzen davon, Amidopropylbetainen und Salzen davon, Alkylhydroxysulfobetainen und Salzen davon, Alkylsulfobetainen und Salzen davon und einer beliebigen Kombination davon.

8. Zusammensetzung gemäß Anspruch 1, wobei das wenigstens eine zwitterionische Tensid oder amphotere Tensid ausgewählt ist aus Cocodimethylcarboxymethylbetain, Lauryldimethylcarboxymethylbetain, Lauryldimethylalpha-carboxyethylbetain, Cetyldimethylcarboxymethylbetain, Laurylbis-(2-hydroxyethyl)carboxymethylbetain, Stearylbis-(2-hydroxypropyl)carboxymethylbetain, Oleyldimethyl-gammacarboxypropylbetain und Laurylbis-(2-hydroxypropyl)-alpha-carboxyethylbetain, Amidopropylbetainen und Alkylsultainen, wie z.B. Cocodimethylsulfopropylbetain, Stearyldimethylsulfopropylbetain, Lauryldimethylsulfoethylbetain, Laurylbis-(2-hydroxyethyl)sulfopropylbetain, Alkylamidopropylhydroxysultainen und einer beliebigen Kombination davon.

9. Zusammensetzung gemäß Anspruch 8, wobei das wenigstens eine zwitterionische Tensid oder amphotere Tensid ein Alkylamidopropylhydroxysultain ausgewählt aus Lauramidopropylhydroxysultain, Laurylhydroxysultain und Cocamidopropylhydroxysultain umfasst.

10. Zusammensetzung gemäß Anspruch 1, wobei die Zusammensetzung ausgewählt ist aus Shampoos, Duschgelen, Handseifen, Lotionen, Cremen, Konditionierern, Rasierprodukten, Gesichtsreinigern, Körperpflegetüchern und Hautbehandlungsmitteln.

11. Zusammensetzung gemäß Anspruch 1, ferner umfassend einen oder mehrere Wirkstoffe.

12. Zusammensetzung gemäß Anspruch 11, wobei der Wirkstoff eine oder mehrere Verbindungen ausgewählt aus Weichmachern, Feuchthaltemitteln, Konditionierungsmitteln, Hautkonditionierungsmitteln, Haarkonditionierungsmitteln, Vitaminen, Provitaminen, Antioxidationsmitteln, Radikalfängern, Abrasivstoffen, Farbstoffen, Haarfärbemitteln, Bleichmitteln, UV-Absorbern, Anti-UV-Mitteln, antibakteriellen Mitteln, antifungiellen Mitteln, Melaninregulatoren, Bräunungsbeschleunigern, Entpigmentierungsmitteln, Hautaufhellern, hautfärbenden Mitteln, Liporegulatoren, Gewichtsreduktionsmitteln, Antiaknemitteln, antiseborrhoischen Mitteln, Antiageingmitteln, Antifaltenmitteln, keratolytischen Mitteln, Entzündungshemmern, Antiaknemitteln, Antibiotika, Entzündungshemmern, Pflanzenextrakten, Imidazolen, Erfrischungsmitteln, narbenbildungsfördernden Mitteln, gefäßschützenden Mitteln, Mitteln zum Verringern von Kopfschuppen, seborrhoischer Dermatitis oder Psoriasis, Schieferöl und Derivaten davon, Antipsoriasismitteln, Corticosteroiden, Enthaarungsmitteln, Mitteln zur Bekämpfung von Haarausfall, Reduktionsmitteln für Dauerwellen, Reflexionsmitteln, ätherischen Ölen und Duftstoffen umfasst.

## Revendications

1. Composition aqueuse de soin personnel sans sulfate, comprenant,
du distéarate de PEG 150,
un composant de type tensioactif comprenant :
(A) au moins un parmi un tensioactif zwitterionique ou un tensioactif amphotère ;
(B) au moins un glycinate d'alcanoyle ; et
(C) de l'acide laurique,
le rapport p/p de A/B étant compris entre 2 et 6 ; et
de l'eau,
la composition comprenant un minimum de 2 % d'acide laurique et 0,1 % à 0,5 % de distéarate de PEG 150 et possédant une plage de pH de 6,5 à 7,0 et une viscosité dans la plage de 2 à 30 Pa·s (2 000 à 30 000 centipoises), mais ne comprenant pas de polyacrylates.

2. Composition selon la revendication 1, le composant de type tensioactif représentant plus de 10 % en poids de la composition de soin personnel.

3. Composition selon la revendication 1, le rapport p/p de A/B étant compris entre 3 et 5, préférablement entre 3,5 et 4,5.

4. Composition selon la revendication 1, le glycinate d'alcanoyle étant de formule (I) : 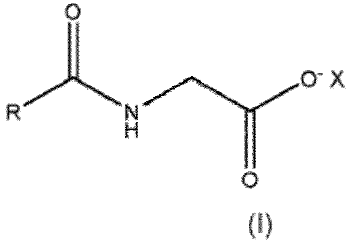 R étant un groupe alkyle en C₈-C₂₂, et X étant un cation ou H.

5. Composition selon la revendication 4, le cation étant choisi parmi sodium, potassium, ou ammonium.

6. Composition selon la revendication 4, R étant un groupe alkyle en C₈-C₁₈, préférablement un groupe alkyle en C₁₀-C₁₄.

7. Composition selon la revendication 1, l'au moins un tensioactif zwitterionique ou tensioactif amphotère étant choisi parmi : des alkylbétaïnes et des sels correspondants, des amidopropylbétaïnes et des sels correspondants, des alkylhydroxysulfobétaïnes et des sels correspondants, des alkylsulfobétaïnes et des sels correspondants, ou une quelconque combinaison correspondante.

8. Composition selon la revendication 1, l'au moins un tensioactif zwitterionique ou tensioactif amphotère étant choisi parmi la cocodiméthylcarboxyméthylbétaïne, la lauryldiméthylcarboxyméthylbétaïne, la lauryldiméthyl-alpha-carboxy-éthylbétaïne, la cétyldiméthylcarboxyméthylbétaïne, la lauryl-bis-(2-hydroxy-éthyl)carboxyméthylbétaïne, la stéarylbis-(2-hydroxy-propyl)carboxyméthylbétaïne, la oléyldiméthylgammacarboxypropylbétaïne, et la lauryl-bis-(2-hydroxypropyl)alpha-carboxyéthylbétaïne, les amidopropylbétaïnes et les alkylsultaïnes, telles que la cocodiméthylsulfopropylbétaïne, la stéaryldiméthylsulfopropylbétaïne, la lauryldiméthylsulfoéthylbétaïne, la lauryl-bis-(2-hydroxy-éthyl)sulfopropylbétaïne, les alkylamidopropylhydroxysultaïnes, ou toute combinaison de celles-ci.

9. Composition selon la revendication 8, l'au moins un tensioactif zwitterionique ou tensioactif amphotère comprenant une alkylamidopropylehydroxysultaïne choisie parmi la lauramidopropylhydroxysultaïne, la laurylhydroxysultaïne et la cocamidopropylhydroxysultaïne.

10. Composition selon la revendication 1, la composition étant choisie parmi des shampooings, des agents de nettoyage corporel, des savons pour les mains, des lotions, des crèmes, des après-shampooings, des produits de rasage, des agents de nettoyage du visage, des lingettes personnelles et des traitements cutanés.

11. Composition selon la revendication 1 comprenant en outre un ou plusieurs agents bénéfiques.

12. Composition selon la revendication 11, l'agent bénéfique comprenant un ou plusieurs composés choisis parmi des émollients, des hydratants, des conditionneurs, des conditionneurs pour la peau, des après-shampooings, des vitamines, des provitamines, des antioxydants, des agents de piégeage de radicaux libres, des agents abrasifs, des colorants, des agents de coloration capillaire, des agents de blanchiment, des absorbants UV, des agents anti-UV, des agents antibactériens, des agents antifongiques, des régulateurs de mélanine, des accélérateurs de bronzage, des agents de dépigmentation, des agents d'éclaircissement de la peau, des agents de coloration de la peau, des liporégulateurs, des agents de réduction du poids, des agents anti-acné, des agents antiséborrhéiques, des agents anti-vieillissement, des agents antirides, des agents kératolytiques, des agents anti-inflammatoires, des agents anti-acné, des antibiotiques, des agents anti-inflammatoires, des extraits botaniques, des imidazoles, des agents rafraîchissants, des agents cicatrisants, des agents de protection vasculaire, des agents pour la réduction des pellicules, de la dermatite séborrhéique, ou du psoriasis, de l'huile de schiste et des dérivés correspondants, des agents anti-psoriasis, des agents de dépilation à base de corticostéroïdes, des agents pour combattre la perte de cheveux, des agents réducteurs pour l'ondulation permanente, des agents de réflectivité, des huiles essentielles et des fragrances.
